# EUROPEAN PATENT APPLICATION

(11) **EP 4 368 116 A1**
(43) Date of publication of application: **15.05.2024**
(21) Application number: 23181305.6
(22) Date of filing: 23.06.2023
(51) Int. Cl.: A61B 8/08, A61B 8/00

(54) **DEVICE FOR ASSISTING IN COLLECTING A BIOLOGICAL SAMPLE FROM A SUBJECT USING A COLLECTION NEEDLE**

(30) Priority: 11.11.2022 PT 2022118336; 20.12.2022 PT 2022118417
(71) Applicant: IPCA - Instituto Politécnico do Cávado e do Ave, 4750-810 V Frescainha (S Martinho) (PT)
(72) Inventor: ARAÚJO MARTINS VILAÇA, JOÃO LUÍS, 4715-566 FRAIÃO (PT); GONÇALVES MORAIS, PEDRO ANDRÉ, 4765-343 OLIVEIRA SANTA MARIA (PT); GOMES OLIVEIRA, BRUNO MIGUEL, 4715-077 LAMAÇÃES (PT); RIBEIRO TORRES, HELENA DANIELA, 4740-043 APÚLIA (PT); DA SILVA VELOSO, FERNANDO JOSÉ, 4730-272 VILA VERDE (PT); FERREIRA REAL, ANTÓNIO, 4750-706 TAMEL - SANTA LEOCÁDIA (PT); STROZS, ROLAND, 4740-593 PALMEIRA DE FARO (PT); VIEIRA FERREIRA DE ARAÚJO, AUGUSTO RIGHETTI, 4750-282 BARCELOS (PT); FERREIRA MATOS, DEMÉTRIO, 4480-395 VILA DO CONDE (PT); MACHADO DE SÁ ABREU TERROSO, MIGUEL, 4760-224 FAMALICÃO (PT)
(74) Representative: Patentree

(57) **Abstract**

The present disclosure relates to a device for assisting in collecting a biological sample from a subject using a collection needle, comprising: a support (1) configured for receiving an ultrasound probe (2); a needle guide (3) for receiving and guiding the collection needle (11); an arm (4) for holding the needle guide (3) to the support (1); a first coupling (5) between the support (1) and the arm (4); a second coupling (6) between the arm (4) and the needle guide (3); wherein the couplings are rotatable couplings, or one of the couplings is a rotatable coupling and another of the couplings is a slidable coupling.

## Description

### TECHNICAL FIELD

The present disclosure relates to a device for assisting in collecting a biological sample from a subject using a collection needle, namely for assisting medical biopsies, as breast, renal and liver biopsies, among others.

### BACKGROUND

Performing a US-guided (ultrasound-guided) biopsy is challenging, an ongoing issue of this procedure is the poor needle visibility, caused by the difficulty of aligning the needle with the ultrasound beam, occurring more frequently with inexperienced doctors [1]. Furthermore, an MRI-detected lesion may be barely visible on the US image considering MRI can detect small lesions. Thus, in recent years several authors are researching techniques to enhance needle visualization in US imaging such as signal/image post-processing, modified needles, robotic assistance, motion analysis and machine learning [2][3].

Image-based needle tracking does not need external hardware as it uses advanced imaging processing algorithms. Most approaches rely on the unprocessed US image containing at least a partially visible needle and line detection techniques are used to detect the needle.

The authors in [4] developed an algorithm that automatically locates the needle in the US image and displays it for the user. To test the algorithm, US images were recorded with the insertion of the needle in an agar phantom to simulate a typical breast biopsy. A group of observers was tasked with recording the values of the angle and tip coordinates of the needle in the image, which was then compared to the results of the algorithm. The algorithm segmentation was precise enough to target 90% of tumors of 4.5mm in diameter.

In [5], the tremors of the hand holding the needle are used to detect the needle location in US-guided interventions. The tremors cause small displacements in the needle and the algorithm subtracts subsequent US images from each other, with the calculated differences providing clues to needle advancement or movement. To evaluate the accuracy of the method, it was tested in vivo on porcine tissue. By comparing the angle of the trajectory calculated by the algorithm to a manual segmentation from an expert, the algorithm had a mean, standard deviation, and RMS error of 2.83º, 1.64º and 3.23º respectively.

Lasers attached to the US probe can improve needle alignment by projecting a line on the skin surface matching the US image plane. In [6]-[8], when puncturing phantoms with the aid of the laser it was verified that the piercing time was lower than free hand. A major limitation of this device however is that it only projects the US plane onto the skin's surface and after the needle has entered the skin it can still leave the plane.

Another technique for easier needle localization is the use of mechanical needle guides that attach to the US probe and work by constraining the needle movement to maintain alignment with the US beam. These needle guides are commercially available and have been shown to improve the success rate and procedure time of percutaneous procedures [9].

There also has been an increase in research into robots capable of performing biopsies in different organs. The use of robots in hospital settings has many benefits such as better accuracy, precision and repeatability of tasks. These robots can usually be categorized by their mechanical design, (how many degrees of freedom (DOFs) they possess), by their mechanical actuation (electrical, pneumatic, cables) and the organ they are meant to target.

In [10] a guidance system for CNB (core needle biopsy) under US guidance was developed comprised of a passive robotic needle and graphical user interfaces for visualizing the needle trajectory. The needle position is tracked using an optical tracking system. The passive robot consists of 5 DOFs, the first and second DOFs are for translation and curvature, the third and fourth DOFs are for rotation motions and the fifth DOF is for needle insertion. The robot is made of polyoxymethylene due to the tensile strength and good impact resistance it offers. The experimental results shown high success rates when performing simulated biopsies and the robot can increase the accuracy and proficiency of needle insertion.

In [11] the authors designed and experimented a fully automated robotic-assisted system for positioning and insertion of a core needle under US guidance. The robotic system consists of a 4 DOF end effector that is attached to a UR5-based teleoperation system with 6 DOF. The robotic system as a whole has the advantages of both freehand and probe-guided interventions. The authors provide a detailed description of the mechanical design as well as a workspace analysis of end effector, which was considered suitable within the US image. Experiments were performed to evaluate the performance of the end effector regarding the needle positioning and insertion in the ultrasound plane. The results showed the ability of the robotic system for in-plane needle insertion with an angle error ranging from 0.148° to 1.011° and a needle displacement error ranging from 0.367 mm to 1.667 mm.

In [12] a group of researchers developed an end-effector for robotic 3D US breast acquisitions and US-guided breast biopsies. The needle guide mechanically guides the needle to a specified lesion within the US plane by using three DOFs to correctly set the trajectory, then the radiologist is responsible for the needle insertion. The needle guide also features a pneumatic mechanism to stop the needle when it reaches the desired depth. The end effector composed of the needle guide and US probe is then mounted on a 7 DOF robotic manipulator. Experiments were conducted to determine the needle placement accuracy of the 3 DOF needle guide and then the biopsy accuracy of the whole system was tested on a cuboid phantom. Needle placement accuracy was 0.3 ± 1.5 mm in and 0.1 ± 0.36 mm out of the US plane. Needle depth was regulated with an accuracy of 100µm. The system reached a Euclidean distance error of 3.21 mm between the needle tip and the target and a normal distance of 3.03 mm between the needle trajectory and the target. The authors concluded the system was capable of assisting the radiologist in breast cancer imaging and biopsy with its high needle placement accuracy.

The available solutions described in the prior art possess several drawbacks, as complexity of use and assembly and/or a lack of versatility to accommodate different ultrasound probes available in the market. Hence, there is a need to provide an improved device for assisting in collecting a biological sample, as a biopsy sample, to surpass the drawbacks identified in the prior art.

These facts are disclosed in order to illustrate the technical problem addressed by the present disclosure.

### GENERAL DESCRIPTION

An aspect of the present disclosure relates to a device for assisting in collecting a biological sample from a subject using a collection needle, comprising:
a support (1) configured for receiving an ultrasound probe (2);
a needle guide (3) for receiving and guiding the collection needle (11);
an arm (4) for holding the needle guide (3) to the support (1);
a first coupling (5) between the support (1) and the arm (4);
a second coupling (6) between the arm (4) and the needle guide (3);
wherein the couplings are rotatable couplings, or
one of the couplings is a rotatable coupling and another of the couplings is a slidable coupling.

In an embodiment, the ultrasound probe (2) has an ultrasound probe axis and the support (1) is configured for receiving an ultrasound probe in a predetermined orientation of the ultrasound probe axis relative to the support.

In an embodiment, the arm (4) is slidably coupled on said support (1).

In an embodiment, the arm (4) is slidable along an axis parallel to the ultrasound probe axis.

In an embodiment, the device may comprise a displacement sensor (7) for measuring displacement of the arm (4) in respect of the support (1).

In an embodiment, the needle guide (3) is rotatably coupled on said arm.

In an embodiment, the device may comprise an angle sensor (8) for measuring rotation between the needle guide (3) and the arm (4).

In an embodiment, the device may comprise a penetration sensor (9) arranged for measuring the translation of a collection needle (11) going through the needle guide (3).

In an embodiment, the needle guide (3) is arranged to rotate around a direction perpendicular to an ultrasound image acquisition plane of the ultrasound probe (2).

In an embodiment, the needle guide (3) is arranged to rotate around within an ultrasound image acquisition plane of the ultrasound probe (2).

In an embodiment, the device may comprise an electronic data processor configured for:
capturing sensor signals from displacement and/or angle sensors, and
processing the captured signals for determining the position and orientation of the needle guide (3) in respect of the support (1), in particular for determining the position and orientation of the needle guide (3) in respect of the ultrasound probe.

In an embodiment, the electronic data processor is further configured for determining the position and orientation of the collection needle (11) in respect of the support (1), in particular for determining the position and orientation of the collection needle (11) in respect of the ultrasound probe (2).

In an embodiment, the needle guide (3) is tubular.

In an embodiment, the support (1) comprises an attachment (10) for attaching the support (1) to an articulated arm (14) or to a robotic arm.

In a preferred embodiment, the electronic data processor is further configured for defining a tool centre point (TCP) in relation to the pre-defined anatomical target (18) and configured for causing rotational movements of an articulated or a robotic arm (14) centred in the lesion TCP.

In an embodiment, the device may comprise a visualisation system configured for displaying an ultrasound image obtained from said ultrasound probe with a representation of the position and orientation of the needle.

In an embodiment, the visualisation system is further configured for providing real-time visual feedback of the pre-defined anatomical target (18), the device, and the TCP, to assist a user in positioning the device accurately.

In an embodiment, the visualisation system is further configured for displaying a collection point at a predetermined distance extending from the collection needle (11), wherein the predetermined distance corresponds to a displacement of a movable collection part of the collection needle (11).

In an embodiment, the device may comprise an electronic data processor configured for:
capturing 2D ultrasound images obtained from said ultrasound probe as the ultrasound probe is moved along the subject;
combining the captured 2D ultrasound images to form a combined 3D ultrasound representation.

In an embodiment, the captured 2D ultrasound images comprise biaxial 2D ultrasound probe images.

In an embodiment, the device may comprise a force sensor for measuring a force applied to the subject by said support (1) and/or said ultrasound probe (2), in particular the force sensor being comprised in a robotic arm or in an attachment to a robotic arm.

In an embodiment, the device may comprise an actuator for applying a predetermined force to the subject by said support (1) and/or said ultrasound probe (2), in particular the actuator being comprised in a robotic arm.

In an embodiment, the device may comprise a robotic arm configured for applying an impedance motion control defined at the tip of the ultrasound probe, in particular the impedance motion control being parameterized for stiffness and damping of the robotic arm.

In an embodiment, the device may comprise an electronic data processor configured for applying an impedance motion control at a robotic arm, defined at the tip of the ultrasound probe, in particular the impedance motion control being parameterized for stiffness and damping of the robotic arm.

In an embodiment, the device may comprise an electronic data processor configured for applying a motion control at a robotic arm for maintaining a predetermined spatial relationship between a centroid of a predetermined anatomical target (18) and an end flange of the robotic arm.

In an embodiment, the electronic data processor is further configured for applying an impedance motion control, in particular being parameterized for stiffness and damping of the robotic arm in respect of orientation and/or position relative to the predetermined anatomical target (18).

In an embodiment, the device may comprise an electronic data processor configured for applying a motion control below a predetermined force maximum applied to the subject at a tip of the ultrasound probe.

### BRIEF DESCRIPTION OF THE DRAWINGS

The following figures provide preferred embodiments for illustrating the disclosure and should not be seen as limiting the scope of invention.
**Figure 1****:** Schematic representation of an embodiment of a device of the present disclosure, and also representing the interaction with an ultrasound probe and a collection needle.
**Figure 2****:** Exploded view of a representation of the device of the present disclosure.
**Figure 3****:** Schematic representation of a device of the present disclosure wherein the support is attached to an articulated arm.
**Figure 4A-4C****:** Representation of the 3D reconstruction around the lesion TCP.

### DETAILED DESCRIPTION

The present disclosure relates to a device for assisting in collecting a biological sample from a subject using a collection needle, comprising: a support (1) configured for receiving an ultrasound probe (2); a needle guide (3) for receiving and guiding the collection needle (11); an arm (4) for holding the needle guide (3) to the support (1); a first coupling (5) between the support (1) and the arm (4); a second coupling (6) between the arm (4) and the needle guide (3); wherein the couplings are rotatable couplings, or one of the couplings is a rotatable coupling and another of the couplings is a slidable coupling.

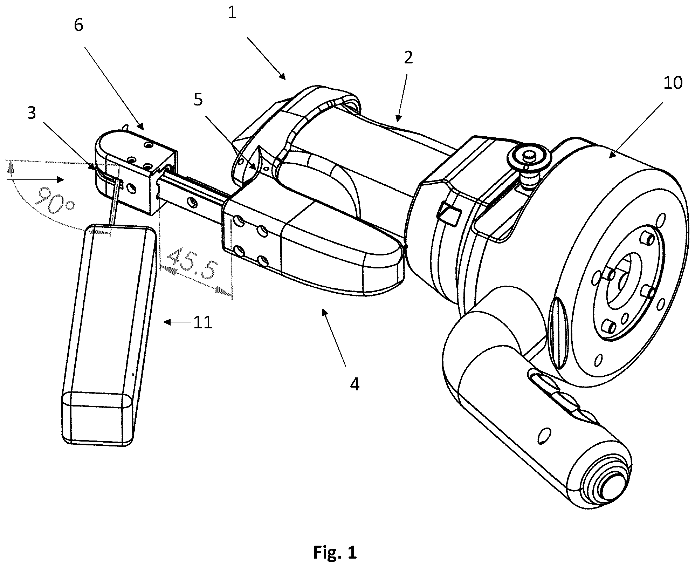

**Figure 1** shows a schematic representation of an embodiment of a device of the present disclosure, and also representing the interaction with an ultrasound probe and a collection needle, wherein:
**1:** support
**2:** ultrasound probe
**3:** needle guide
**4:** arm
**5:** first coupling
**6:** second coupling
**10:** attachment
**11:** collection needle

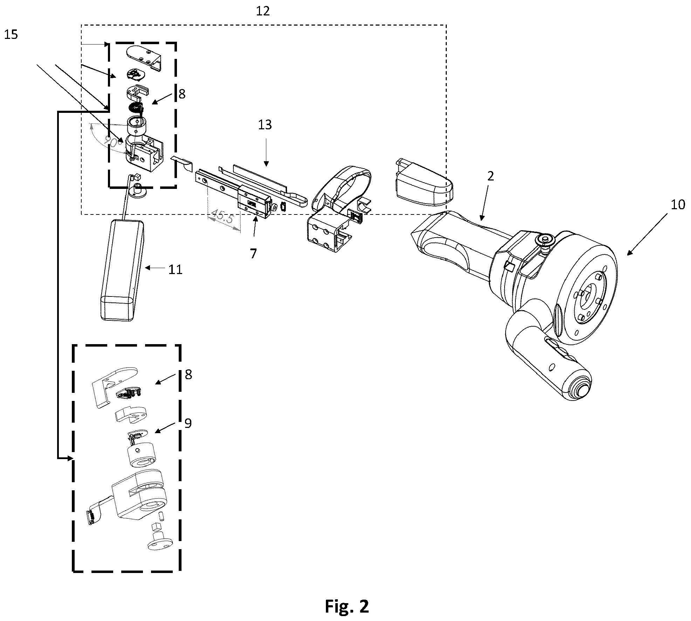

**Figure 2** shows an exploded view of a representation of the device of the present disclosure, wherein:
**2:** ultrasound probe
**7:** displacement sensor
**8:** angle sensor
**9:** penetration sensor
**10:** attachment
**11:** collection needle
**12:** device of the present disclosure
**13:** magnetic band
**15:** mechanical adapters

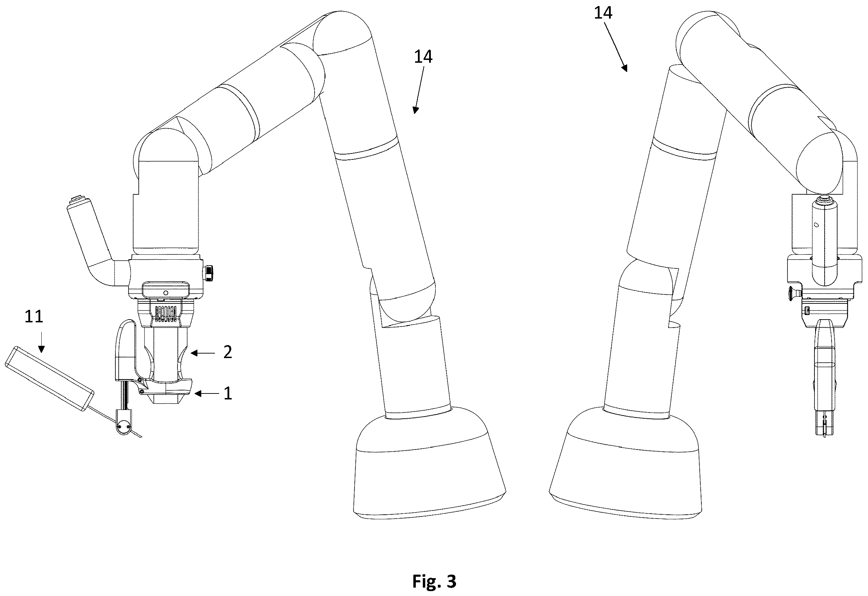

**Figure 3** shows a schematic representation of a device of the present disclosure wherein the support is attached to an articulated arm, wherein:
**1:** support
**2:** ultrasound probe
**11:** collection needle
**14:** articulated arm

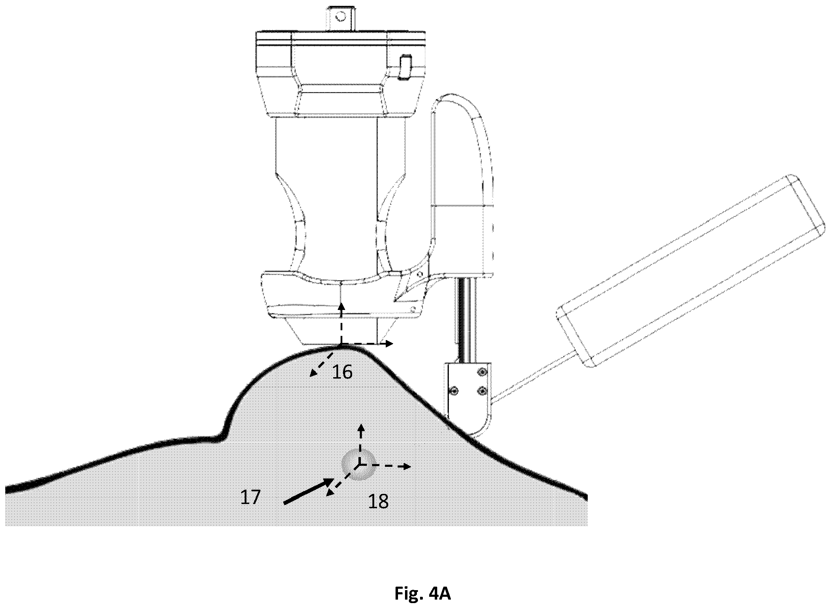

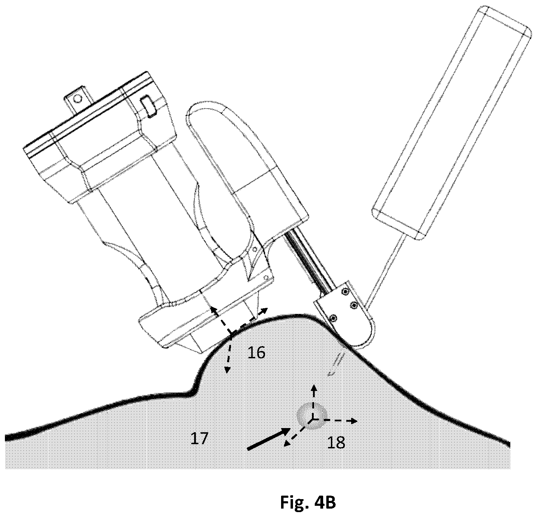

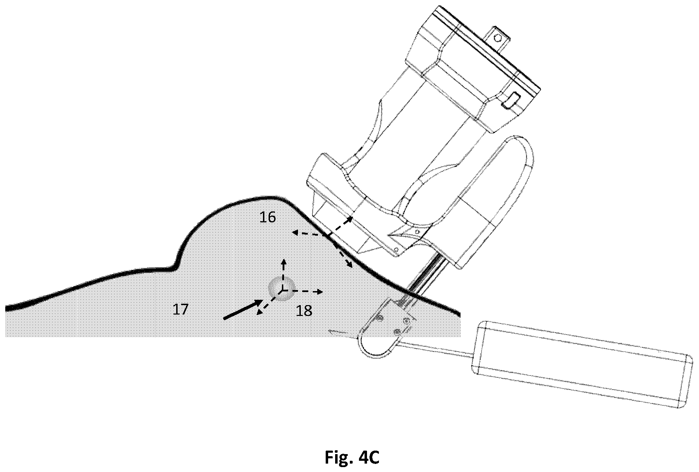

Figure 4A-C shows a representation of the 3D reconstruction around the lesion TCP, wherein:
**16:** TCP tip
**17:** breast lesion
**18:** TCP lesion (anatomical target)

In an embodiment, the device of the present disclosure allows to align the collection needle with the ultrasound image acquisition plane.

In an embodiment, the needle guide is a mechanical structure with at least 2 degrees of freedom.

In an embodiment, the needle guide is tubular and the tubular guide is used to restrict the penetration of the collection needle with the ultrasound image plane.

In an embodiment, the tubular guide has a length of 25 mm.

In an embodiment, the needle guide may be sensorized to infer the angle and penetration angle of the needle (collection needle) in relation to the origin of the ultrasound image.

In an embodiment, the displacement sensor is, for example, a AS5600 (AMS).

In an embodiment, the angle sensor is, for example, AS5311 (AMS)

In an embodiment, the needle penetration sensor is, for example, PAT9125 (Pixart Imaging).

In an embodiment, the arm has an extension length of 45.5 mm and the needle guide is rotatable with a mechanical limit of 90 °.

In an embodiment, the ultrasound image acquisition system is in contact with the tissues of a subject.

In an embodiment, the visualization system has capability to create an augmented visualization of the ultrasound image, enhancing the angle and position of the needle in relation to the anatomical target. The visualization system may also have the capability to enhance the needle position and virtually represent the movable structure of the biopsy needle (collection needle).

The visualization system may display different visualization information (e.g. color, tracing) to be used to confirm the correct penetration of the needle in relation to the centroid of the target lesion.

In an embodiment, the support comprises an attachment for attaching the support to an articulated arm with a set of axes that immobilizes in a pre-defined position the device.

In an embodiment, the articulated arm may be a robotic arm with at least 6DOF.

In an embodiment, the articulated robotic arm may have a torque sensor in each joint or a force sensor at the robotic base, joint or tip. The articulated robotic arm attached to the support may autonomously maintain the applied force, throughout the remaining stages of the treatment.

In an embodiment, the operator moves the articulated arm for a pre-defined position, which is in contact with the tissue, applying a force for the ultrasound image acquisition.

In an embodiment, the applied force may be defined by the operator.

In an embodiment, the applied force may be defined according to the guidelines.

The articulated robotic may have an impedance motion control defined at the tip of the ultrasound probe parameterized by the stiffness and the damping. The articulated robotic arm dynamically may adapt the stiffness to maintains the recorded applied force. The stiffness is defined by the Hooke Law computed as the ratio between force and displacement.

In an embodiment, the damping coefficient is defined to guarantee a smooth movement of the articulated robotic arm.

The tool centre point (TCP) is the point used to move a robot arm to a Cartesian position (such as a Cartesian target given XYZWPR values). The TCP is usually defined as a transformation from the robot flange.

In a preferred embodiment, the tool centre point (TCP) of the articulated arm is dynamically defined at the centroid of a pre-defined anatomical target. The TCP relation describes spatial relation between the centroid of the anatomical target and the tip of the robotic arm. In this way, all motions of the articulated arm are always referenced with the anatomical target.

The pre-defined anatomical target can be defined automatically (using an artificial intelligent method that detect the lesion on ultrasound Image and later estimates its 3D centroid) or manually (by the operator selecting the desired position on the ultrasound image).

In an embodiment, the pre-defined anatomical target is not superficial and it is visible on the ultrasound image.

In an embodiment, the articulated robotic arm automatically dynamics the pose of the support (1) to minimizes the error between the initial TCP relation and the current one, while keeping constant the applied force on the tissue. In this way, the articulated arm with the compliant concept suppresses on the ultrasound images eventual movements caused by the respiratory movements or involuntary movements, keeping the anatomical target in the same site.

In an embodiment, 3D reconstruction of the anatomical target can be performed by defining a set of trajectories around the TCP. For that, rotational movements of the articulated arm centred in the lesion TCP are performed. In each movement, a 2D ultrasound image is acquired. The rotational information and the multiple 2D ultrasound planes are combined to reconstruct the 3D volume.

In embodiment, the device of the present disclosure can be used in medical biopsies, namely ultrasound-guided renal biopsies, ultrasound-guided liver biopsies, ultrasound-guided breast biopsies, ultrasound-guided supra-renal biopsies, ultrasound-guided prostate biopsies, ultrasound-guided thyroid biopsies; preferably ultrasound-guided breast biopsies.

In an embodiment, the needle guide can be positioned automatically, ensuring that the needle trajectory during the biopsy is the same as the predefined trajectory.

The needle guide may further comprise motors, encoders, displacement sensors, controllers to mechanically actuate each axis.

In an embodiment, trajectory of the needle (collection needle) may be pre-defined in the visualization system or automatically through an optimization process that minimizes the penetration distance and limits the entry angle, according to a pre-defined maximum value, of the needle. The pre-defined maximum entry angle can be defined according to the medical guidelines.

A joystick may be used to operate the articulated arm.

To perform a refined assessment of the region to be biopsied, a joystick may be used to automatically adjust the articulated arm position and pose.

In an embodiment, the joystick automatically modifies the anatomical TCP position.

The joystick can be a 6DOF mouse or a haptic feedback device.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above described embodiments are combinable.

The following claims further set out particular embodiments of the disclosure.

### References

[1] B. D. Sites, B. C. Spence, J. D. Gallagher, C. W. Wiley, M. L. Bertrand, and G. T. Blike, "Characterizing Novice Behavior Associated With Learning Ultrasound-Guided Peripheral Regional Anesthesia," Reg Anesth Pain Med, vol. 32, no. 2, pp. 107-115, Mar. 2007, doi: 10.1016/J.RAPM.2006.11.006.
[2] P. Beigi, S. E. Salcudean, G. C. Ng, and R. Rohling, "Enhancement of needle visualization and localization in ultrasound," International Journal of Computer Assisted Radiology and Surgery, vol. 16, no. 1. Int J Comput Assist Radiol Surg, pp. 169-178, Jan. 01, 2021. doi: 10.1007/s11548-020-02227-7.
[3] H. J. Scholten, A. Pourtaherian, N. Mihajlovic, H. H. M. Korsten, and R. A. Bouwman, "Improving needle tip identification during ultrasound-guided procedures in anaesthetic practice," Anaesthesia, vol. 72, no. 7, pp. 889-904, Jul. 2017, doi: 10.1111/ANAE.13921.
[4] K. J. Draper, C. C. Blake, L. Gowman, D. B. Downey, and A. Fenster, "An algorithm for automatic needle localization in ultrasound-guided breast biopsies," Med Phys, vol. 27, no. 8, pp. 1971-1979, Aug. 2000, doi: 10.1118/1.1287437.
[5] P. Beigi, • Robert Rohling, S. E. Salcudean, • Gary, and C. Ng, "Spectral analysis of the tremor motion for needle detection in curvilinear ultrasound via spatiotemporal linear sampling," Int J CARS, vol. 11, pp. 1183-1192, 2016, doi: 10.1007/s11548-016-1402-7.
[6] B. C. H. Tsui, "Facilitating needle alignment in-plane to an ultrasound beam using a portable laser unit," Reg Anesth Pain Med, vol. 32, no. 1, pp. 84-88, Jan. 2007, doi: 10.1016/J.RAPM.2006.09.009.
[7] S. Y. Liu et al., "Laser assisted ultrasound guided aspiration improves procedure time and reduces number of withdrawals," Ultrasonics, vol. 48, no. 8, pp. 647-651, Dec. 2008, doi: 10.1016/J.ULTRAS.2008.03.002.
[8] G. B. Collins, E. M. Fanou, J. Young, and P. Bhogal, "A comparison of free-hand vs laser-guided long-axis ultrasound techniques in novice users," Br J Radiol, vol. 86, no. 1029, Sep. 2013, doi: 10.1259/BJR.20130026.
[9] J. R. England, T. Fischbeck, and H. Tchelepi, "The Value of Needle-Guidance Technology in Ultrasound-Guided Percutaneous Procedures Performed by Radiology Residents: A Comparison of Freehand, In-Plane, Fixed-Angle, and Electromagnetic Needle Tracking Techniques," Journal of Ultrasound in Medicine, vol. 38, no. 2, pp. 399-405, Feb. 2019, doi: 10.1002/jum.14701.
[10] N. Tanaiutchawoot, B. Treepong, and J. Suthakorn, "On the Design of A Biopsy Needle-Holding Robot for A Novel Breast Biopsy Robotic Navigation System Cholatip Wiratkapan (Medical Consultant)," 2014.
[11] S. M. Sajadi, S. M. Karbasi, H. Brun, J. Tørresen, O. J. Elle, and K. Mathiassen, "Towards Autonomous Robotic Biopsy-Design, Modeling and Control of a Robot for Needle Insertion of a Commercial Full Core Biopsy Instrument," Front Robot Al, vol. 9, Jun. 2022, doi: 10.3389/frobt.2022.896267.
[12] M. K. Welleweerd, F. J. Siepel, V. Groenhuis, J. Veltman, and S. Stramigioli, "Design of an end-effector for robot-assisted ultrasound-guided breast biopsies," Int J Comput Assist Radiol Surg, vol. 15, no. 4, pp. 681-690, Apr. 2020, doi: 10.1007/s11548-020-02122-1.

## Claims

1. Device for assisting in collecting a biological sample from a subject using a collection needle, comprising:
a support (1) configured for receiving an ultrasound probe (2);
a needle guide (3) for receiving and guiding the collection needle (11);
an arm (4) for holding the needle guide (3) to the support (1);
a first coupling (5) between the support (1) and the arm (4);
a second coupling (6) between the arm (4) and the needle guide (3);
wherein the couplings are rotatable couplings, or
one of the couplings is a rotatable coupling and another of the couplings is a slidable coupling.

2. Device according to the previous claim wherein the ultrasound probe (2) has an ultrasound probe axis and the support (1) is configured for receiving an ultrasound probe in a predetermined orientation of the ultrasound probe axis relative to the support.

3. Device according to any of the previous claims wherein the arm (4) is slidably coupled on said support (1); preferably the arm (4) is slidable along an axis parallel to the ultrasound probe axis.

4. Device according to any of the claims 2-3 comprising a displacement sensor (7) for measuring displacement of the arm (4) in respect of the support (1) and/or comprising an angle sensor (8) for measuring rotation between the needle guide (3) and the arm (4).

5. Device according to any of the previous claims wherein the needle guide (3) is rotatably coupled on said arm; preferably the needle guide (3) is arranged to rotate around within an ultrasound image acquisition plane of the ultrasound probe (2).

6. Device according to the previous claim wherein the needle guide (3) is arranged to rotate around a direction perpendicular to an ultrasound image acquisition plane of the ultrasound probe (2); preferably the needle guide (3) is tubular.

7. Device according to any of the previous claims comprising a penetration sensor (9) arranged for measuring the translation of a collection needle (11) going through the needle guide (3).

8. Device according to any of the previous claims comprising an electronic data processor configured for:
capturing sensor signals from displacement and/or angle sensors, and
processing the captured signals for determining the position and orientation of the needle guide (3) in respect of the support (1), in particular for determining the position and orientation of the needle guide (3) in respect of the ultrasound probe.

9. Device according to the previous claim wherein the electronic data processor is further configured for determining the position and orientation of the collection needle (11) in respect of the support (1), in particular for determining the position and orientation of the collection needle (11) in respect of the ultrasound probe (2).

10. Device according to any of the previous claims wherein the support (1) comprises an attachment (10) for attaching the support (1) to an articulated arm (14) or to a robotic arm.

11. Device according to any of the previous claims 8 or 9 wherein the electronic data processor is further configured for defining a tool centre point (TCP) in relation to the pre-defined anatomical target (18) and configured for causing rotational movements of an articulated or a robotic arm (14) centred in the anatomical target.

12. Device according to any of the previous claims comprising a visualisation system configured for displaying an ultrasound image obtained from said ultrasound probe with a representation of the position and orientation of the needle; preferably the visualisation system is further configured for providing real-time visual feedback of the pre-defined anatomical target (18), the device, and the TCP, to assist a user in positioning the device accurately.

13. Device according to the previous claim wherein the visualisation system is further configured for displaying a collection point at a predetermined distance extending from the collection needle (11), wherein the predetermined distance corresponds to a displacement of a movable collection part of the collection needle (11).

14. Device according to the claim 15 or 16 comprising an electronic data processor configured for:
capturing 2D ultrasound images obtained from said ultrasound probe as the ultrasound probe is moved along the subject;
combining the captured 2D ultrasound images to form a combined 3D ultrasound representation;
preferably the captured 2D ultrasound images comprise biaxial 2D ultrasound probe images.

15. Device according to any of the previous claims comprising a force sensor for measuring a force applied to the subject by said support (1) and/or said ultrasound probe (2), in particular the force sensor being comprised in a robotic arm or in an attachment to a robotic arm; and/ or comprising an actuator for applying a predetermined force to the subject by said support (1) and/or said ultrasound probe (2), in particular the actuator being comprised in a robotic arm; and/or comprising a robotic arm configured for applying an impedance motion control defined at the tip of the ultrasound probe, in particular the impedance motion control being parameterized for stiffness and damping of the robotic arm.
